# FASCICULE DE BREVET EUROPEEN

(11) **EP 1 661 975 B1**
(45) Date de publication et mention de la délivrance du brevet: **02.07.2008**
(21) Numéro de dépôt: 05292171.5
(22) Date de dépôt: 14.10.2005
(51) Int. Cl.: C11D 1/02, C11D 3/37, A61Q 19/10, A61K 8/81

(54) **Composition liquide de nettoyage à base de tensioactifs anioniques; utilisations pour le nettoyage des matières keratiniques kératiniques humaines**
Flüssiges Reinigungsmittel auf Basis von anionischen Tensiden und deren Verwendung zur Haarwäsche
Liquid cleaning composition comprising anionic surfactant and its use for hair cleaning

(30) Priorité: 26.11.2004 FR 0452780
(43) Date de publication de la demande: 31.05.2006
(73) Titulaire: L'ORÉAL, 75008 Paris (FR)
(72) Inventeur: Bissey-Beugras, Laure, 92300 Levallois-Perret (FR); Ribery, Delphine, 75013 Paris (FR)
(74) Mandataire: Miszputen, Laurent

(56) Documents cités:
- EP-A- 0 013 836
- EP-A- 0 459 077
- WO-A-20/05023969
- US-A- 5 507 971
- US-A1- 2003 103 926
- US-A1- 2003 108 503
- US-A1- 2003 147 842

## Description

La présente invention a trait à une composition liquide de nettoyage, caractérisée par le fait qu'elle contient dans un milieu aqueux cosmétiquement acceptable:
(a) au moins un tensioactif anionique ;
(b) au moins un électrolyte.
(c) au moins un copolymère d'acide méthacrylique et d'acrylate d'alkyle en C₁-C₄, réticulé dans une quantité inférieure à 0,5% en matière active en poids par rapport au poids total de la composition.
(d) éventuellement au moins un tensioactif choisi parmi les tensioactifs non-ioniques, amphotères et zwitterioniques;
(e) la quantité totale de tensioactif(s) en poids de matière active étant supérieure à 5% en poids.

La présente invention concerne également ses utilisations dans les domaines cosmétique ou dermatologique, notamment comme produits de nettoyage des matières kératiniques humaines et plus particulièrement de la peau.

Les compositions liquides lavantes pour la douche, appelées généralement "gels douche" "sont particulièrement appréciées par les consommateurs. Ils sont en général sous forme de lait, de crème ou de gel crème conditionnés en tube, en flacon, flacon-pompe, en mousse aérosol. Ces formulations comprennent cinq grandes familles de formulation détergente : (1) celles à base de lauryl sulfate ; celles à base d'alpha-oléfine sulfonate, (3) celles à base de mélange de tensio-actifs synthétiques anioniques, amphotères et/ou non-ioniques ; (4) les formulations à base de savons ; (5) les formulations mixtes à bases de savons et de tensio-actifs synthétiques.

Ces compositions ont été décrites notamment dans les documents suivants :
- US 4,387,040 ; US 4,310,432 et US 310,433.
- Formulation Technology of Liquid Soaps by Eugene M. Franck - Cosmetic & Toiletries Vol 97, 1982, pages 49-54.
- Liquid Soap, a challenge for the formulator by Roger Hart - Household & Personal Care Products Industry May 1981, pages 46-47 ;
- Comparison of Detergent Based versus Soap Based Liquid Soaps by Dennis W. Dyer and Thomas Hassapis- Soap/Cosmetics/Chemical Specialities for July , 1993pages 36; 38 and 40 ;
- The Evolution of Liquid Soap by Larry Lundmark - Cosmetic & Toiletries Vol 107, December 1992, pages 49-52.

Les formulations liquides pour la douche sont particulièrement recherchées pour les avantages suivants:
- ils ont de bonnes propriétés lavantes et produisent en général une mousse crémeuse, aérée et dense, qui démarre rapidement,
- ils sont hygiéniques par leur conditionnement,
- ils sont faciles à transporter par leur conditionnement ;
- leur utilisation est simple et économique ;
- ils laissent la peau douce et non tiraillée après application et rinçage.

Ces compositions de nettoyage pour le corps ou les mains selon le conditionnement doivent présenter une consistance et une rhéologie appropriées pour une bonne appréhension du produit par l'utilisateur et un bon étalement sur la surface de la peau à laver. Elles contiennent en général un système épaississant choisis par exemple parmi les électrolytes comme le chlorure de sodium, le chlorure de potassium ou le sulfate de potassium ; les alcanolamides comme le cocamide DEA ou le cocamide MEA ; les esters de polyethylèneglycol et de monoacide ou diacide stéarique comme le distéarate de polyéthylèneglycol 6000 ou leurs mélanges ; les polymères épaississants comme les polymères acryliques réticulés comme les « Carbomer » ou les copolymères du type acrylates/C₁₀-C₃₀alkylacrylate (CARBOPOL 1382).

Certains des gels douche actuellement sur le marché en raison de la présence de certains épaississants pose des difficultés de fabrication. C'est le cas notamment des épaississants comme les « Carbomer » ou les copolymères du type acrylates/C₁₀₋C₃₀alkylacrylate sous forme de poudre. Ces derniers nécessitent lors de la fabrication industrielle des gels douche le contenant, d'être incorporés dans l'eau froide et d'être mélangés suffisamment longtemps pour être complètement homogénéisés dans la phase aqueuse.

Pour surmonter ces problèmes de mise en oeuvre, une solution consiste à utiliser comme système épaississant un électrolyte. Cependant, la demanderesse a constaté que certains gels douches obtenus avec ce type d'épaississant pouvaient être sensibles aux basses températures (en dessous de 20°C) et que la texture du produit était substantiellement altérée. Cette thermosensibilité se traduit par une texture élastique et non-filante à la sortie du conditionnement qui rend l'étalement difficile : le produit se casse en morceaux qui glissent sur la peau mouillée.

Il subsiste donc le besoin de rechercher une composition liquide pour la toilette du corps à base de tensioactifs détergents et d'un système épaississant appropriés dont la fabrication industrielle ne présente pas de difficultés de mise en oeuvre et dont la texture ne présente pas les inconvénients décrits. Le système épaississant et la base lavante utilisés ne doivent pas altérer les propriétés lavantes ni la qualité de la mousse comme le démarrage de la mousse ni affecter les propriétés cosmétiques du produit comme la sensation de douceur sur la peau après rinçage.

On connaît dans l'état de la technique des compositions liquides détergentes contenant un copolymère d'acide méthacrylique et d'acrylate d'alkyle comme agent de stabilisation ou de suspension d'ingrédients insolubles dans l'eau comme des silicones, des corps gras ou des agents nacrant. De telles compositions ont été décrites dans les demandes de brevets JP 3-34915, JP 4-169519 et WO01/76552. Les qualités de mousse et les propriétés cosmétiques obtenues avec ces compositions ne sont pas encore suffisamment satisfaisantes.

On connaît dans l'exemple 11 de la demande WO2005/023969 une formulation détergente ayant la composition suivante :

| **Ingrédient** | **Nom INCI** | **Quantité en % en poids** |
|---|---|---|
| CARBOPOL AQUA SF-1 (30%) | Acrylates copolymer | 0.900 |
| ATLAS G-4280 (72%) | PEG-80 Sorbitan Laurate | 4.580 |
| TEGOBETAINE L7V (30%) | Cocoamidopropyl Betaine | 11.330 |
| GLYCERIN 917 (99%) | Glycerin | 1.900 |
| POLYMER JR-400 | Polyquaternium-10 | 0.140 |
| DOWICIL 200 | Quaternium-15 | 0.050 |
| VERSENE 100 XL | Tetrasodium EDTA | 0.263 |
| EAU | Eau | qs 100 |

Ce document n'évoque que le problème technique de réduire l'irritation de la peau et/ou des yeux.

La demanderesse a découvert de manière surprenante que les problèmes techniques énoncés précédemment pouvaient être résolus en utilisant (1) un système détergent constitué d'au moins un tensioactif anionique, (2) au moins un système épaississant comprenant (i) au moins un électrolyte et (ii) au moins un copolymère réticulé d'acide méthacrylique et d'acrylate d'alkyle en C₁-C₄ dans une quantité inférieure à 0,5% en poids en matière active par rapport au poids total de la composition.

La présente invention a donc pour objet une composition liquide de nettoyage, caractérisée par le fait qu'elle contient dans un milieu aqueux cosmétiquement acceptable:
(a) au moins un tensioactif anionique ;
(b) au moins un électrolyte.
(c) au moins un copolymère réticulé d'acide méthacrylique et d'acrylate d'alkyle en C₁-C₄, dans une quantité inférieure à 0,5% en matière active en poids par rapport au poids total de la composition :
(d) éventuellement au moins un tensioactif choisi parmi les tensioactifs non-ioniques, amphotères et zwitterioniques;
(e) la quantité totale de tensioactif(s) en poids de matière active étant supérieure à 5% en poids; sous réserve que la composition soit différente de la formulation telle que définie dans le tableau suivant :

| **Ingrédient** | **Nom INCI** | **Quantité en % en poids** |
|---|---|---|
| CARBOPOL AQUA SF-1 (30%) | Acrylates copolymer | 0.900 |
| ATLAS G-4280 (72%) | PEG-80 Sorbitan Laurate | 4.580 |
| TEGOBETAINE L7V (30%) | Cocoamidopropyl Betaine | 11.330 |
| GLYCERIN 917 (99%) | Glycerin | 1.900 |
| POLYMER JR-400 | Polyquaternium-10 | 0.140 |
| DOWICIL 200 | Quaternium-15 | 0.050 |
| VERSENE 100 XL | Tetrasodium EDTA | 0.263 |
| EAU | Eau | qs 100 |

Un autre objet de l'invention consiste en l'utilisation cosmétique de la composition telle que définie ci-dessus, comme produits de nettoyage et/ou de démaquillage des matières kératiniques.

Un autre objet de l'invention consiste en un procédé cosmétique de nettoyage des résidus de salissure des matières kératiniques humaines, caractérisé par le fait qu'on applique la composition de l'invention, sur lesdites matières kératiniques, en présence d'eau, qu'on masse pour former une mousse et qu'on élimine la mousse formée et les résidus de salissure par rinçage à l'eau.

La présente invention concerne aussi l'utilisation d'un copolymère réticulé d'acide méthacrylique et d'acrylate d'alkyle en C₁-C₄ tel que défini précédemment à un taux inférieur à 0,5% en poids en matière active dans une composition liquide de nettoyage contenant dans un milieu aqueux cosmétiquement acceptable, au moins un tensioactif anionique et au moins un électrolyte, dans le but d'améliorer la stabilité de la texture de ladite composition à basse température.

Dans toute la description, on entend par « matières kératiniques humaines » la peau (corps ou visage y compris les mains, les lèvres, les paupières et le cuir chevelu), les ongles et les phanères comme les cheveux, les cils et les sourcils.

On entend ici par « milieu physiologiquement acceptable » un milieu compatible avec la peau, les muqueuses, le cuir chevelu, les yeux et/ou les cheveux. Par ailleurs, il s'agit d'un milieu aqueux, c'est-à-dire d'un milieu comportant de l'eau et de préférence une quantité d'eau d'au moins 35 % en poids, par exemple allant de 35 à 95 % en poids et mieux de 40 à 80 % en poids par rapport au poids total de la composition.

Les compositions de l'invention sont des compositions de nettoyage liquides, appropriées pour une application topique, et notamment pour une application sur la peau. Elles se présentent généralement sous forme d'un gel pouvant s'écouler ou non sous son propre poids, c'est-à-dire ayant une viscosité pouvant aller par exemple de 5 poises à 250 poises (0,5 à 25 Pa.s) et de préférence de 35 poises à 200 poises (3,5 à 20 Pa.s), la viscosité étant mesurée à 25°C avec un appareil de mesure Rheomat 180 à 200 s-1, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités supérieures à 20 poises.

L'une des caractéristiques essentielles de l'invention est la présence d'un copolymère réticulé d'acide méthacrylique et d'acrylate d'alkyle en C₁-C₄.

L'acide méthacrylique est présent de préférence dans des quantités allant de 20 à 80% en poids et plus particulièrement de 25 à 70 % en poids et encore plus particulièrement de 35 à 65% en poids par rapport au poids total du copolymère.

L'acrylate d'alkyle est présent de préférence dans des quantités allant de 15 à 80% en poids et plus particulièrement de 25 à 75 % en poids et encore plus particulièrement de 35 à 65% en poids par rapport au poids total du copolymère. Il est choisi notamment parmi l'acrylate de méthyle, l'acrylate d'éthyle ou l'acrylate de butyle et plus particulièrement l'acrylate d'éthyle.

Ce copolymère est de préférence partiellement ou totalement réticulé par au moins un agent réticulant classique éthylèniquement polyinsaturé comme les polyalcényléthers de sucrose ou de polyols, les diallylphtalates, le divinylbenzene, le (meth)acrylate d'allyle, di(meth)acrylate d'éthyleneglycol, le méthylène bis-acrylamide, le tri(meth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés . La teneur en agent réticulant varie en général de 0,01 à 5% en poids et de préférence de 0,03 à 3% en poids et encore plus particulièrement de 0,05 à 1% en poids par rapport au poids total du copolymère.

Selon une forme particulièrement préférée, le copolymère de l'invention peut se présenter notamment sous forme de dispersion dans l'eau. La taille moyenne des particules de copolymère dans la dispersion est généralement comprise entre 10 et 500 nm et de préférence entre 20 et 200 nm et plus préférentiellement de 50 à 150 nm.

La concentration en copolymère ne doit pas dépasser la valeur de 0,5% en poids en matière active. Au delà de cette concentration, la qualité de la mousse commence à être altérée de manière sensible notamment au niveau du démarrage de la mousse et de sa quantité.

La concentration en copolymère varie de préférence de 0,01 et 0,4% en poids en matière active par rapport au poids total de la composition et de préférence de 0,1 à 0,3 % en poids.

L'électrolyte présent dans la composition est choisi de préférence parmi les sels de métal alcalin comme le chlorure de sodium, le chlorure de potassium ou le sulfate de potassium et plus particulièrement le chlorure de sodium. Il sera de préférence utilisé à des concentrations supérieures ou égales à 0,25% en poids par rapport au poids total de la composition et plus préférentiellement allant 0,25 à 5 % en poids par rapport et plus particulièrement de 0,5 à 3%.

Les compositions selon l'invention contiennent au moins un tensioactif anionique.

On peut utiliser par exemple comme tensioactifs anioniques, les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

Comme carboxylates, on peut citer par exemple :
- les amido éthercarboxylates (AEC), comme le lauryl amido ether carboxylate de sodium (3 OE) commercialisé sous la dénomination AKYPO FOAM 30® par la société Kao Chemicals ;
- les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxyméthyle, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX ® par la société Nikkol ;
- les acétates tels que le 2-(2-hydroxy alkyloxy)acétate de sodium commercialisé sous la dénomination BEAULIGHT SHAA par la société Sanyo ;
- les sels alcalins de N-acylaminoacides, comme par exemple (1) les sarcosinates, comme le lauroyl sarcosinate de sodium commercialisé sous la dénomination SARKOSYL NL 97® par la société Ciba ou sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; (2) les alaninates comme le N-lauroyl-N-methylamidopropionate de sodium, commercialisé sous la dénomination SODIUM NIKKOL ALANINATE LN 30® par la société Nikkol ou sous la dénomination ALANONE ALE® par la société Kawaken, et le N-lauroyl N-methylalanine triéthanolamine, commercialisé sous la dénomination ALANONE ALTA® par la société Kawaken ; (3) les acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; (4) les aspartates comme le mélange de N-lauroylaspartate de triéthanolamine et de N-myristoylaspartate de triéthanolamine, commercialisé sous la dénomination ASPARACK® par la société Mitsubishi ; (5) les glycinates comme le N-cocoyl glycinate de sodium commercialisé sous les dénominations AMILITE GCS-12® et AMILITE GCK 12 par la société Ajinomoto ;
- les citrates tels que le mono-ester citrique d'alcools de coco oxyéthylénés (9 OA) commercialisé sous la dénomination WITCONOL EC 1129 par la société Goldschmidt ;
- les galacturonates comme le Dodecyl D- galactoside uronate de sodium commercialisé par la société SOLIANCE ;
- les savons qui sont des sels d'acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides, et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

Comme alkyl sulfates oxyéthylénés ou non, on peut citer par exemple le lauryl éther sulfate de sodium (C12-14 70/30) (2,2 OE) commercialisé sous la dénomination SIPON AOS 225® par la société Cognis, le lauryl éther sulfate d'ammonium (C12-14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le mélange de lauryl et oleyl éther sulfate de sodium et magnésium commercialisé sous la dénomination EMPICOL BSD 52 par la société Albright & Wilson.

Comme sulfonates, on peut citer par exemple (1) les alpha-oléfines sulfonates comme l'alpha-oléfine sulfonate de sodium (C14-16) commercialisé sous la dénomination BIO-TERGE AS-40 pa s dénominations BIO-TERGE AS-40® et dénomination BIO-TERGE AS-40® par la société Stepan, sous les dénominations WITCONATE AOS PROTEGE® et SULFRAMINE AOS PH 12® par la société Witco, l'oléfine sulfonate de sodium secondaire commercialisé sous la dénomination HOSTAPUR SAS 30® par la société Clariant ; (2) les iséthionates comme le cocoyl-iséthionate de sodium, tel que le produit commercialisé sous la dénomination JORDAPON CI P® par la société Jordan, (3) les taurates comme le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo.

Comme phosphates, on peut citer par exemple les monoalkylphosphates et les dialkylphosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea.

Comme polypeptides (qui sont des composés obtenus par condensation d'une chaîne grasse sur les aminoacides de céréale et notamment du blé et de l'avoine), on peut citer par exemple le sel de potassium de la lauroyl protéine de blé hydrolysée, commercialisé sous la dénomination AMINOFOAM W OR® par la société Croda ; le sel de triéthanolamine de cocoyl protéine de soja hydrolysée, commercialisé sous la dénomination MAY-TEIN SY® par la société Maybrook ; le sel de sodium des lauroyl amino-acides d'avoine, commercialisé sous la dénomination PROTEOL OAT® par la société Seppic ; l'hydrolysat de collagène greffé sur l'acide gras de coprah, commercialisé sous la dénomination GELIDERM 3000® par la société Deutsche Gélatine ; les protéines de soja acylées par des acides de coprah hydrogénés, commercialisées sous la dénomination PROTEOL VS 22® par la société Seppic.

Comme dérivés anioniques d'alkyl-polyglucosides, on peut citer notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia ; le sel di-sodique d'ester sulfosuccinique de cocoylpolyglucoside (1,4), commercialisé sous la dénomination ESSAI 512 MP® par la société Seppic ; le sel de sodium d'ester citrique de cocoyl polyglucoside (1,4) commercialisé sous la dénomination EUCAROL AGE-EC® par la société Cesalpinia, le Lauryl polyglucoside éther carboxylate de sodium commercialisé sous la dénomination PLANTAPON LGC SORB par la société Cognis.

On utilisera de préférence les sels d'alkyléthersulfates en C₆-C₂₄ comportant de 1 à 30 groupements oxyde d'éthylène , en particulier les sels de métaux alcalins ou alcalino-terreux, d'ammonium, d'amine ou d'aminoalcool et plus particulièrement les sels de sodium et encore plus particulièrement les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate (nom CTFA).

Selon une forme particulièrement préférée de l'invention, les compositions contiennent en plus au moins un tensio-actif amphotère ou zwitterionique.

On peut utiliser par exemple comme tensioactifs amphotères et zwitterioniques, les alkylbétaïnes, les N-alkylamidobétaïnes et leurs dérivés, les sultaïnes, les alkyl-polyaminocarboxylate (APAC), les alkylamphoacétates et leurs mélanges.

Comme alkylbétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme sultaines, on peut citer le cocoyl-amidopropylhydroxy-sulfobetaine commercialisé sous la dénomination CROSULTAINE C-50® par la société Croda.

Comme alkyl-polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel ; le stéaryl-polyamidocarboxylate de sodium commercialisé sous la dénomination AMPHOLAK 7 TX/C® par la société Akzo Nobel ; la carboxyméthyloléyl-polypropylamine de sodium, commercialisé sous la dénomination AMPHOLAK X07/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacetate).

On utilisera plus particulièrement parmi les tensio-actifs amphotères
- la cocobétaine tels que les produits commerciaux MIRATAINE BB/FLA de RHODIA ou EMPIGEN BB/FL de Huntsman.
- le cocoamphodiacétate de disodium comme le produit commercialisé sous la dénomination commerciale MIRANOL® C2M concentré par la société RHODIA. ou leurs mélanges.

Les compositions selon l'invention peuvent contenir en plus un ou plusieurs tensioactifs non ioniques. Ce sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkylphénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant de préférence en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de préférence de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀₋C₁₄)amines ou les oxydes de N-acyl(C₁₀-C₁₄)-aminopropylmorpholine ; et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Cognis, et sous la dénomination ORAMIX NS 10® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que I'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

La composition de nettoyage selon l'invention constitue une composition moussante et détergente, et elle contient au moins un tensioactif anionique qui va apporter le caractère moussant à la composition. Elle peut également contenir en plus d'autres tensioactifs non ioniques, amphotères et zwitterioniques.

La quantité totale de tensioactif(s) en poids de matière active est supérieure à 5 % en poids. Elle peut aller de préférence de 5 à 50 % en poids, plus préférentiellement de 6 à 50 % en poids, mieux de 6 à 30 % en poids et encore mieux de 8 à 25 % en poids par rapport au poids total de la composition.

Le milieu aqueux cosmétiquement acceptable des compositions de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition. De préférence, la composition comprend de 50 à 95 % en poids d'eau par rapport au poids total de la composition.

Les compositions de l'invention contiennent de préférence en plus un ou plusieurs polymères cationiques du type polyquaternium, qui apportent douceur et onctuosité à la composition moussante.

Ces polymères cationiques peuvent être choisis de préférence parmi les polymères suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 10 tel que le produit Polymer JR400 commercialisé par la société AMERCHOL;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Les compositions de l'invention peuvent comprendre des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques tels que le polyvinylpyrrolidone (PVP), les polymères anioniques, les corps gras incompatibles avec le milieu aqueux, comme les huiles ou les cires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme exemple d'huile, on peut citer les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

Comme actifs, on peut utiliser dans la composition de l'invention tout actif habituellement utilisé dans les domaines cosmétique et dermatologique, tels que par exemple les vitamines ou provitamines hydrosolubles ou liposolubles comme les vitamines A (rétinol), C (acide ascorbique), B3 ou PP (niacinamide), B5 (panthénol), E (tocophérol), K1, le bêta-carotène, et les dérivés de ces vitamines et notamment leurs esters ; les stéroïdes comme la DHEA et la 7α-hydroxy DHEA ; les antiseptiques ; les antisébohrréïques et antimicrobiens tels que le peroxyde de benzoyle, l'acide salicylique, le triclosan, le tricarban, l'acide azélaïque ; les hydratants comme la glycérine, l'acide hyaluronique, le pyrrolidone carboxylique acide (PCA) et ses sels, le pidolate de sodium, la sérine, le xylitol, le tréhalose, l'ectoïne, les céramides, l'urée ; les agents kératolytiques et anti-âge tels que les alpha-hydroxyacides comme l'acide glycolique, l'acide citrique, l'acide lactique, les béta-hydroxyacides comme l'acide salicylique et ses dérivés ; les enzymes et co-enzymes et notamment le coenzyme Q10 ; les filtres solaires ; les azurants optiques ; les actifs amincissants comme la caféine, la théophyline, la théobromine, les anti-inflammatoires tels que les acides 18 β glycyrrhétinique et ursolique, et leurs mélanges. On peut utiliser un mélange de deux ou plusieurs de ces actifs. Le ou les actifs peuvent être par exemple présents en une concentration allant de 0,01 à 20 %, de préférence de 0,1 à 10 % et mieux de 0,5 à 5% du poids total de la composition.

Comme charges, on peut citer les charges minérales telles que le talc ou silicate de magnésium (granulométrie: 5 microns) commercialisé sous la dénomination LUZENAC 15 M00® par la société Luzenac, le kaolin ou silicate d'aluminium comme par exemple celui commercialisé sous la dénomination KAOLIN SUPREME® par la société Imerys, ou les charges organiques telles que l'amidon comme par exemple le produit commercialisé sous la dénomination AMIDON DE MAIS B ® par la société Roquette, les microsphères de Nylon comme celles commercialisées sous la dénomination ORGASOL 2002 UD NAT COS® par la société Atochem, les microsphères à base de copolymère de chlorure de vinylidene/Acrylonitrile/methacrylonitrile enfermant de l'isobutane, expansées comme celles commercialisées sous la dénomination EXPANCEL 551 DE® par la société Expancel. On peut ajouter aussi à la composition de l'invention, des fibres comme par exemple des fibres de nylon (POLYAMIDE 0.9 DTEX 0.3 MM commercialisé par les Etablissements Paul Bonté), des fibres de cellulose ou « Rayonne » (RAYON FLOCK RCISE NOOO3 MO4® commercialisé par la société Claremont Flock Corporation).

Comme agents nacrants ou opacifiants, on peut citer les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Les compositions moussantes selon l'invention peuvent être utilisées dans les domaines cosmétique et dermatologique, et elles peuvent constituer notamment une composition cosmétique et en particulier des produits de nettoyage ou de démaquillage de la peau (corps, visage, yeux), du cuir chevelu et/ou des cheveux. Elles peuvent être utilisées pour tout type de peau (sèche, normale, mixte ou grasse).

Les compositions de l'invention peuvent notamment être utilisées comme produit pour la douche ; produit pour le bain ; comme produit de nettoyage des mains ; comme shampooing ; comme produit démaquillant des yeux et/ou du visage.

Un autre objet de l'invention consiste en l'utilisation cosmétique de la composition telle que définie ci-dessus, comme produit de nettoyage et/ou de démaquillage des matières kératiniques humaines et plus particulièrement de la peau.

Les compositions selon l'invention peuvent être aussi utilisées pour le traitement des peaux grasses, notamment en y ajoutant des actifs spécifiques de traitement des peaux grasses, tels que les antiséborrhéiques comme par exemple l'acide salicylique et ses dérivés, l'acide azélaïque, le triclosan, le tricarban, le piroctone olamine, la niacinamide (vitamine PP).

Un autre objet de l'invention est l'utilisation de la composition telle que définie ci-dessus pour la préparation d'une composition destinée au traitement des peaux grasses.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions de l'invention peuvent être conditionnées selon l'application choisie sous forme de flacon, de tube, de flacon-pompe, de dispositif aérosol ou tout autre mode de conditionnement approprié pour une composition liquide de lavage.

Les compositions de nettoyage de la présente invention conditionnées dans des dispositifs aérosols peuvent contenir n'importe quel agent propulseur usuellement employé pour la préparation de compositions aérosols. On peut citer en particulier les gaz hydrocarbonés, comme par exemple le propane, le n-butane, l'isobutane et leurs mélanges ; les gaz fluorés comme par exemple le chlorodifluorométhane, le dichlorodifluorométhane, le difluoroéthane, le chlorodifluoroéthane, le dichlorotétrafluoroéthane, etc. et leurs mélanges ; les gaz hydrofluorocarbonés ; le dimethyl ether et les mélanges de dimethyl ether avec un ou plusieurs gaz hydrocarbonés ; l'azote, l'air et le dioxyde de carbone et leurs mélanges peuvent également être utilisés comme gaz propulseurs dans la présente invention. De façon préférentielle, on utilise dans la présente invention des gaz hydrocarbonés ayant de 2 à 6 atomes de carbone et plus particulièrement un mélange iso-butane, propane, n-butane. Le ou les gaz propulseurs sont présents dans le dispositif à raison de 0,1 à 15% en poids et plus préférentiellement de 1 à 8% en poids par rapport au poids total de la composition.

La présente invention concerne enfin l'utilisation d'un copolymère d'acide méthacrylique et d'acrylate d'alkyle en C₁-C₄, réticulé ou non-réticulé dans une composition liquide de nettoyage contenant dans un milieu aqueux cosmétiquement acceptable, au moins un tensioactif anionique et au moins un électrolyte, dans le but d'améliorer la stabilité de la texture de ladite composition à basse température.

Les exemples qui suivent servent à illustrer l'invention sans toutefois présenter un caractère limitatif. Les quantités indiquées sont en % en poids sauf mention contraire, et les noms des composés en noms chimiques ou noms CTFA (International Cosmetic Ingredient Dictionary and Handbook) selon les cas.

| | **Exemple 1 *(% en poids MA)*** | **Exemple 2 *(% en poids MA)*** | **Exemple 3 *(% en poids MA)*** | **Exemple 4 *(% en poids MA)*** |
|---|---|---|---|---|
| | **Invention** | **Invention** | **Hors Invention** | **Hors invention** |
| **SODIUM LAURETH SULFATE** | 9,80 | 9,80 | 9,80 | 9,80 |
| **SODIUM CHLORIDE** | 2,16 | 2,16 | 2,16 | 2,16 |
| **COCO-BETAINE** | 1,9875 | 1,9875 | 1,9875 | 1,9875 |
| **GLYCERIN** | 1 | 1 | 1 | 1 |
| **HEXYLENE GLYCOL** | 1 | 1 | 1 | 1 |
| **GLYCOL DISTEARATE** | 1 | 1 | 1 | 1 |
| **DISODIUM COCOAMPHODIACETATE** | 0,6 | 0,6 | 0,6 | 0,6 |
| **DISODIUM EDTA** | 0,26 | 0,26 | 0,26 | 0,26 |
| **ACRYLATES COPOLYMER** | 0,24 | 0,399 | 0,6 | 0 |
| **SODIUM GLYCOLATE** | 0,12 | 0,12 | 0,12 | 0,12 |
| **POLYQUATERNIUM-7** | 0,11 | 0,11 | 0,11 | 0,11 |
| **CONSERVATEURS** | qs | qs | qs | qs |
| **PARFUM** | qs | qs | qs | qs |
| **EAU** | 82,8122 | 82,6532 | 82,4522 | 83,0522 |

La composition est préparée selon le mode opératoire suivant :
1) Dans la cuve de fabrication :
   - Introduire l'eau, les conservateurs et la glycérine.
   - Ajouter sous agitation plus lente, une partie de sodium laureth sulfate jusqu'à complète solubilisation.
   - Ajouter ensuite successivement, sous agitation, le disodium EDTA, le polyquaternium-7, l'acrylates copolymer et le parfum.
2) Faire fondre le glycol distéarate avec une partie de l'eau et du sodium laureth sulfate.
3) Ajouter cette préparation puis la coco bétaïne et le disodium ecocoamphodiacétate dans la cuve de fabrication.

Elle présente de bonnes propriétés moussantes et lavantes ; elle ne dessèche pas la peau et laisse une sensation de douceur après rinçage. On observe aucune texture élastique à la sortie du conditionnement lorsque l'on stocke la composition à une température de l'ordre 15°C -18°C.

L'observation de la texture élastique est faite d'u point de vue sensoriel, comme décrit ci-dessous :
- Peser 2 g de produit dans une coupelle et la placer à 4°C pendant 15 minutes.
- A la sortie tapoter avec une spatule : si l'aspect est élastique et gélatineux, la texture du produit n'est pas acceptable.

Cette observation a été faite pour les formules correspondants aux exemples 1 à 4 :
Les exemples 1 et 2 contenant le copolymère d'acrylate dans une quantité inférieure à 0,5% en poids en matière active présentent une texture acceptable, l'aspect dans la coupelle n'est ni élastique, ni gélatineux mais filant.
L'exemple 3 comparatif contenant le copolymère d'acrylate dans une quantité supérieure à 0,5% en poids a des qualités cosmétiques non satisfaisantes notamment le démarrage et la quantité de la mousse.
L'exemple 4 comparatif ne contenant pas le copolymère d'acrylate présente une texture pas acceptable, l'aspect dans la coupelle est élastique et gélatineux.

## Revendications

1. Composition liquide de nettoyage, **caractérisée par le fait qu'**elle contient dans un milieu aqueux cosmétiquement acceptable :
(a) au moins un tensioactif anionique ;
(b) au moins un électrolyte;
(c) au moins un copolymère réticulé d'acide méthacrylique et d'acrylate d'alkyle en C₁-C₄ dans une quantité inférieure à 0,5% en matière active en poids par rapport au poids total de la composition ;
(d) éventuellement au moins un tensioactif choisi parmi les tensioactifs non-ioniques, amphotères et zwitterioniques ; (e) la quantité totale de tensioactif(s) en poids de matière active étant supérieure à 5% en poids ; sous réserve que la composition soit différente de la formulation telle que définie dans le tableau suivant :
| **Ingrédient** | **Nom INCI** | **Quantité en % en** poids |
|---|---|---|
| CARBOPOL AQUA SF-1 (30%) | Acrylates copolymer | 0.900 |
| ATLAS G-4280 (72%) | PEG-80 Sorbitan Laurate | 4.580 |
| TEGOBETAINE L7V (30%) | Cocoamidopropyl Betaine | 11.330 |
| GLYCERIN 917 (99%) | Glycerin | 1.900 |
| POLYMER JR-400 | Polyquaternium-10 | 0.140 |
| DOWICIL 200 | Quaternium-15 | 0.050 |
| VERSENE 100 XL | Tetrasodium EDTA | 0.263 |
| EAU | Eau | qs 100 |

2. Composition selon la revendication 1, où dans ledit copolymère, l'acide méthacrylique est présent dans des quantités allant de 20 à 80% en poids et plus particulièrement de 25 à 70 % en poids et encore plus particulièrement de 35 à 65% en poids par rapport au poids total du copolymère.

3. Composition selon la revendication 1 ou 2, où dans ledit copolymère, l'acrylate d'alkyle est présent dans des quantités allant de 15 à 80% en poids et plus particulièrement de 25 à 75 % en poids et encore plus particulièrement de 35 à 65% en poids par rapport au poids total du copolymère.

4. Composition selon l'une quelconque des revendications 1 à 3, où dans ledit copolymère, l'acrylate d'alkyle est choisi parmi l'acrylate de méthyle, l'acrylate d'éthyle ou l'acrylate de butyle

5. Composition selon la revendication 4, où l'acrylate d'alkyle est l'acrylate d'éthyle.

6. Composition selon l'une quelconque des revendications 1 à 5, où le copolymère copolymère d'acide méthacrylique et d'acrylate d'alkyle en C₁-C₄ est partiellement ou totalement réticulé par au moins un agent réticulant classique éthylèniquement polyinsaturé.

7. Composition selon la revendication 6, où la teneur en agent réticulant varie de 0,01 à 5% en poids et de préférence de 0,03 à 3% en poids et encore plus particulièrement de 0,05 à 1% en poids par rapport au poids total du copolymère.

8. Composition selon l'une quelconque des revendications 1 à 5, où le copolymère copolymère d'acide méthacrylique et d'acrylate d'alkyle en C₁-C₄ est sous forme de dispersion de particules dans l'eau.

9. Composition selon la revendication 8, où la taille moyenne des particules de copolymère dans la dispersion varie de 10 à 500 nm et de préférence de 20 à 200 nm et plus préférentiellement de 50 à 150 nm.

10. Composition selon l'une quelconque des revendications 1 à 9 où le copolymère copolymère d'acide méthacrylique et d'acrylate d'alkyle en C₁-C₄ est le copolymère acide méthacryliquelacrylate d'éthyle réticulé sous forme de dispersion aqueuse à 30%.

11. Composition selon l'une quelconque des revendications 1 à 9, où le copolymère copolymère d'acide méthacrylique et d'acrylate d'alkyle en C₁-C₄ est présent dans une concentration allant de 0,01 et 0,4% en poids en matière active par rapport au poids total de la composition et de préférence de 0,1 à 0,3 % en poids.

12. Composition selon l'une quelconque des revendications 1 à 11, où l'électrolyte est choisi parmi les sels de métal alcalin.

13. Composition selon la revendication 12, où l'électrolyte est le chlorure de sodium, le chlorure de potassium ou le sulfate de potassium.

14. Composition selon l'une quelconque des revendications 1 à 13, où l'électrolyte est présent à des concentrations supérieures ou égales à 0,25% par rapport au poids total de la composition et plus particulièrement de 0,5 à 3% en poids.

15. Composition selon la revendication 14, où l'électrolyte est présent à des concentrations allant 0,25 à 5 % en poids par rapport au poids total de la composition et plus particulièrement de 0,5 à 3% en poids.

16. Composition selon l'une quelconque des revendications 1 à 15, où le tensioactif anionique est choisi parmi les carboxylates, les alkyl sulfates oxyéthylénés ou non ; les sulfonates ; les alkyl sulfoacétates ; les phosphates ; les polypeptides ; les dérivés anioniques d'alkyl polyglucoside et leurs mélanges.

17. Composition selon la revendication 16, où le tensioactif anionique est choisi parmi les sels d'alkyléthersulfates en C₆-C₂₄ comportant 1 à 30 groupements oxyde d'éthylène

18. Composition selon la revendication 17, où le tensioactif anionique est choisi parmi les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate.

19. Composition selon l'une quelconque des revendications 1 à 18, comprenant en plus au moins un tensioactif amphotère ou zwitterionique.

20. Composition selon la revendication 19, où le tensioactif amphotère ou zwitterionique est choisi parmi les alkylbétaïnes, les N-alkylamidobétaïnes et leurs dérivés, les sultaïnes, les alkyl-polyaminocarboxylate, les alkylamphoacétates et leurs mélanges.

21. Composition selon la revendication 20, où le tensioactif amphotère ou zwitterionique est choisi parmi
- la cocobétaine,
- le cocoamphodiacétate de disodium ou leurs mélanges.

22. Composition selon l'une quelconque des revendications 1 à 21**,** comprenant en plus au moins un tensioactif non ionique.

23. Composition selon la revendication 22, où le tensioactif non-ionique est choisi parmi
- les alcools, alpha-diols, alkylphénols ou acides gras polyéthoxylés, polypropoxylés ou polyglycérolés ayant une chaîne grasse ;
- les copolymères d'oxyde d'éthylène et de propylène,
- les condensats d'oxyde d'éthylène et de propylène sur des alcools gras ;
- les amides gras polyéthoxylés,
- les amides gras polyglycérolés,
- les amines grasses polyéthoxylées,
- les esters d'acides gras du sorbitane éthoxylés
- les esters d'acides gras du saccharose,
- les esters d'acides gras du polyéthylèneglycol,
- les alkyl(C6-C24)polyglycosides,
- les dérivés de N-alkyl(C6-C24)glucamine,
- les oxydes d'amines,
- les oxydes de N-acyl(C₁₀-C₁₄)-aminopropylmorpholine et leurs mélanges.

24. Composition selon l'une quelconque des revendications 1 à 22, où la quantité totale de tensioactif(s) en poids de matière active est varie de 5 à 50 % en poids, de préférence de 6 à 50 % en poids, mieux de 6 à 30 % en poids et encore mieux de 8 à 25 % en poids par rapport au poids total de la composition.

25. Composition selon l'une quelconque des revendications 1 à 24, comprenant en plus un ou plusieurs polymères cationiques.

26. Composition selon l'une quelconque des revendications 1 à 25, comprenant en plus 1 un ou plusieurs adjuvants cosmétiques choisis parmi es parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques, les polymères anioniques, les corps gras.

27. Utilisation cosmétique d'une composition telle que définie dans l'une quelconque des revendications 1 à 26, comme produit de nettoyage et/ou de démaquillage des matières kératiniques humaines.

28. Utilisation selon la revendication 27, comme produit pour le bain ou la douche.

29. Utilisation selon la revendication 27, comme produit pour le nettoyage des mains.

30. Utilisation selon la revendication 27, comme shampooing.

31. Utilisation selon la revendication 27, comme produit démaquillant des yeux, du visage ou des lèvres.

32. Utilisation de la composition telle que définie dans l'une quelconque des revendications 1 à 26, pour la préparation d'une formulation destinée au traitement des peaux grasses.

33. Procédé cosmétique de nettoyage des résidus de salissure des matières kératiniques humaines, **caractérisé par le fait qu'**on applique une composition telle que définie dans l'une quelconque des revendications 1 à 26 sur lesdites matières kératiniques, en présence d'eau, qu'on masse pour former une mousse et qu'on élimine la mousse formée et les résidus de salissure par rinçage à l'eau.

34. Utilisation d'un copolymère réticulé d'acide méthacrylique et d'acrylate d'alkyle en C₁-C₄, tel que défini dans l'une quelconque des revendications 1 à 10 à un taux inférieur à 0,5% en poids en matière active dans une composition liquide de nettoyage telle que définie dans les revendications 1 à 26 dans le but d'améliorer la stabilité de la texture de ladite composition à une température inférieure à 20°C.

## Claims

1. Liquid cleansing composition, **characterized in that** it contains, in a cosmetically acceptable aqueous medium:
(a) at least one anionic surfactant;
(b) at least one electrolyte,
(c) at least one crosslinked copolymer of methacrylic acid and of a C₁-C₄ alkyl acrylate, in an amount of less than 0.5o by weight of active material relative to the total weight of the composition;
(d) optionally at least one surfactant chosen from nonionic, amphoteric and zwitterionic surfactants;
(e) the total amount of surfactant(s) by weight of active material being greater than 5% by weight;
with the proviso that the composition is not the same as the formulation as defined in the following table:
| **Ingredient** | **INCI name** | **Amount in weight %** |
|---|---|---|
| Carbopol Aqua SF-1 (30%) | Acrylates copolymer | 0.900 |
| Atlas G-4280 (72%) | PEG-80 sorbitan laurate | 4.580 |
| Tegobetaine L7V (30%) | Cocoamidopropyl betaine | 11.330 |
| Glycerin 917 (99%) | Glycerin | 1.900 |
| Polymer JR-400 | Polyquaternium-10 | 0.140 |
| Dowicil 200 | Quaternium-15 | 0.050 |
| Versene 100 XL | Tetrasodium EDTA | 0.263 |
| Water | Water | qs 100 |

2. Composition according to Claim 1, in which, in the said copolymer, the methacrylic acid is present in amounts ranging from 20% to 80% by weight, more particularly from 25% to 70% by weight and even more particularly from 35% to 65% by weight relative to the total weight of the copolymer.

3. Composition according to Claim 1 or 2, in which, in the said copolymer, the alkyl acrylate is present in amounts ranging from 15% to 80% by weight, more particularly from 25% to 75% by weight and even more particularly from 35% to 65% by weight relative to the total weight of the copolymer.

4. Composition according to any one of Claims 1 to 3, in which, in the said copolymer, the alkyl acrylate is chosen from methyl acrylate, ethyl acrylate or butyl acrylate.

5. Composition according to Claim 4, in which the alkyl acrylate is ethyl acrylate.

6. Composition according to any one of Claims 1 to 5, in which the copolymer of methacrylic acid and of a C₁-C₄ alkyl acrylate is partially or totally crosslinked with at least one standard polyethylenically unsaturated crosslinking agent.

7. Composition according to Claim 6, in which the content of crosslinking agent ranges from 0.01% to 5% by weight, preferably from 0.03% to 3% by weight and even more particularly from 0.05% to 1% by weight relative to the total weight of the copolymer.

8. Composition according to any one of Claims 1 to 5, in which the copolymer of methacrylic acid and of a C₁-C₄ alkyl acrylate is in the form of a dispersion of particles in water.

9. Composition according to Claim 8, in which the mean size of the copolymer particles in the dispersion ranges from 10 to 500 nm, preferably from 20 to 200 nm and more preferentially from 50 to 150 nm.

10. Composition according to any one of Claims 1 to 9, in which the copolymer of methacrylic acid and of a C₁-C₄ alkyl acrylate is the crosslinked methacrylic acid/ethyl acrylate copolymer in the form of an aqueous 30% dispersion.

11. Composition according to any one of Claims 1 to 9, in which the copolymer of methacrylic acid and of a C₁-C₄ alkyl acrylate is present in a concentration ranging from 0.01% to 0.4% by weight and preferably from 0.1% to 0.3% by weight of active material relative to the total weight of the composition.

12. Composition according to any one of Claims 1 to 11, in which the electrolyte is chosen from alkali metal salts.

13. Composition according to Claim 12, in which the electrolyte is sodium chloride, potassium chloride or potassium sulfate.

14. Composition according to any one of Claims 1 to 13, in which the electrolyte is present in concentrations of greater than or equal to 0.25% and more particularly from 0.5% to 3% by weight relative to the total weight of the composition.

15. Composition according to Claim 14, in which the electrolyte is present in concentrations ranging from 0.25% to 5% by weight and more particularly from 0.5% to 3% by weight relative to the total weight of the composition.

16. Composition according to any one of Claims 1 to 15, in which the anionic surfactant is chosen from carboxylates, oxyethylenated or non-oxyethylenated alkyl sulfates; sulfonates; alkyl sulfoacetates; phosphates; polypeptides; anionic derivatives of alkyl polyglucoside, and mixtures thereof.

17. Composition according to Claim 16, in which the anionic surfactant is chosen from C₆-C₂₄ alkyl ether sulfate salts containing from 1 to 30 ethylene oxide groups.

18. Composition according to Claim 17, in which the anionic surfactant is chosen from oxyethylenated sodium (C₁₂-C₁₄)alkyl ether sulfates comprising a mean number of ethylene oxide groups of between 1 and 4, and more particularly sodium laureth sulfate.

19. Composition according to any one of Claims 1 to 18, also comprising at least one amphoteric or zwitterionic surfactant.

20. Composition according to Claim 19, in which the amphoteric or zwitterionic surfactant is chosen from alkylbetaines, N-alkylamidobetaines and derivatives thereof, sultaines, alkylpolyaminocarboxylates and alkylamphoacetates, and mixtures thereof.

21. Composition according to Claim 20, in which the amphoteric or zwitterionic surfactant is chosen from:
- cocobetaine,
- disodium cocoamphodiacetate or mixtures thereof.

22. Composition according to any one of Claims 1 to 21, also comprising at least one nonionic surfactant.

23. Composition according to Claim 22, in which the nonionic surfactant is chosen from:
- polyethoxylated, polypropoxylated or polyglycerolated fatty alcohols, fatty α-diols, fatty alkylphenols or fatty acids, containing a fatty chain;
- copolymers of ethylene oxide and of propylene oxide,
- condensates of ethylene oxide and of propylene oxide with fatty alcohols;
- polyethoxylated fatty amides,
- polyglycerolated fatty amides,
- polyethoxylated fatty amines,
- ethoxylated fatty acid esters of sorbitan,
- fatty acid esters of sucrose,
- fatty acid esters of polyethylene glycol,
- (C₆-C₂₄) alkylpolyglycosides,
- N-(C₆-C₂₄)alkylglucamine derivatives,
- amine oxides,
- N- (C₁₀-C₁₄) acylaminopropylmorpholine oxides,
and mixtures thereof.

24. Composition according to any one of Claims 1 to 22, in which the total amount of surfactant(s) by weight of active material ranges from 5% to 50% by weight, preferably from 6% to 50% by weight, better still from 6% to 30% by weight and even better still from 8% to 25% by weight relative to the total weight of the composition.

25. Composition according to any one of Claims 1 to 24, also comprising one or more cationic polymers.

26. Composition according to any one of Claims 1 to 25, also comprising one or more cosmetic adjuvants chosen from fragrances, preserving agents, sequestering agents (EDTA), pigments, nacreous agents or opacifiers, mineral or organic fillers, matting agents, bleaching agents or exfoliants, soluble dyes, cosmetic or dermatological active agents, nonionic polymers, anionic polymers and fatty substances.

27. Cosmetic use of a composition as defined in any one of Claims 1 to 26, as a product for cleansing and/or removing makeup from human keratin materials.

28. Use according to Claim 27, as a bath or shower product.

29. Use according to Claim 27, as a product for cleansing the hands.

30. Use according to Claim 27, as a shampoo.

31. Use according to Claim 27, as a product for removing makeup from the eyes, the face or the lips.

32. Use of the composition as defined in any one of Claims 1 to 26, for the preparation of a formulation for treating greasy skin.

33. Cosmetic process for cleansing soiling residue from the human keratin materials, **characterized in that** a composition as defined in any one of Claims 1 to 26 is applied to the said keratin materials, in the presence of water, it is massaged to form a lather and the lather formed and the soiling residue are removed by rinsing with water.

34. Use of a crosslinked copolymer of methacrylic acid and of a C₁-C₄ alkyl acrylate as defined in any one of Claims 1 to 10, in a proportion of less than 0.5% by weight of active material in a liquid cleansing composition as defined in Claims 1 to 26, for the purpose of improving the stability of the texture of the said composition at a temperature below 20°C.

## Patentansprüche

1. Flüssige Reinigungszusammensetzung, **dadurch gekennzeichnet, dass** sie in einem kosmetisch akzeptablen, wässrigen Medium enthält:
(a) mindestens einen anionischen grenzflächenaktiven Stoff;
(b) mindestens einen Elektrolyten;
(c) mindestens ein vernetztes Copolymer von Methacrylsäure und C₁₋₄-Alkylacrylat in einem Mengenanteil unter 0,5 Gew.-% wirksame Substanz, bezogen auf das Gesamtgewicht der Zusammensetzung;
(d) gegebenenfalls mindestens einen grenzflächenaktiven Stoff, der unter den nichtionischen, amphoteren und zwitterionischen grenzflächenaktiven Stoffen ausgewählt ist;
(e) wobei die Gesamtmenge an Tensid(en), ausgedrückt als Gewicht der wirksamen Substanz, über 5 Gew.-% liegt;
mit der Maßgabe, dass die Zusammensetzung von der in der folgenden Tabelle definierten Formulierung verschieden ist:
| **Bestandteil** | **INCI-Name** | **Mengenanteil in Gew.-%** |
|---|---|---|
| CARBOPOL AQUA SF-1 (30 %) | Acrylates Copolymer | 0,900 |
| ATLAS G-4280 (72 %) | PEG-80 Sorbitan Laurate | 4,580 |
| TEGOBETAINE L7V (30 %) | Cocoamidopropyl Betaine | 11,330 |
| GLYCERIN 917 (99 %) | Glycerin | 1,900 |
| POLYMER JR-400 | Polyquaternium-10 | 0,140 |
| DOWICIL 200 | Quaternium-15 | 0,050 |
| VERSENE 100 XL | Tetrasodium EDTA | 0,263 |
| EAU | Eau | qs 100 |

2. Zusammensetzung nach Anspruch 1, wobei in dem Copolymer die Methacrylsäure in Mengenanteilen von 20 bis 80 Gew.-% und insbesondere 25 bis 70 Gew.-% und besonders 35 bis 65 Gew.-%, bezogen auf das Gesamtgewicht des Copolymers, enthalten ist.

3. Zusammensetzung nach Anspruch 1 oder 2, wobei in dem Copolymer das Alkylacrylat in Mengenanteilen von 15 bis 80 Gew.-% und insbesondere 25 bis 75 Gew.-% und besonders 35 bis 65 Gew.-%, bezogen auf das Gesamtgewicht des Copolymers, enthalten ist.

4. Zusammensetzung nach einem der Ansprüche 1 bis 3, wobei in dem Copolymer das Alkylacrylat unter Methylacrylat, Ethylacrylat oder Butylacrylat ausgewählt ist.

5. Zusammensetzung nach Anspruch 4, wobei das Alkylacrylat das Ethylacrylat ist.

6. Zusammensetzung nach einem der Ansprüche 1 bis 5, wobei das Copolymer von Methacrylsäure und C₁₋₄-Alkylacrylat ganz oder teilweise mit mindestens einem herkömmlichen, mehrfach ethylenisch ungesättigten Vernetzungsmittel vernetzt ist.

7. Zusammensetzung nach Anspruch 6, wobei der Mengenanteil des Vernetzungsmittels im Bereich von 0,01 bis 5 Gew.-%, vorzugsweise 0,03 bis 3 Gew.-% und insbesondere 0,05 bis 1 Gew.-%, bezogen auf das Gesamtgewicht des Copolymers, liegt.

8. Zusammensetzung nach einem der Ansprüche 1 bis 5, wobei das Copolymer von Methacrylsäure und C₁₋₄-Alkylacrylat in Form einer Dispersion von Partikeln in Wasser vorliegt.

9. Zusammensetzung nach Anspruch 8, wobei die mittlere Größe der Copolymerpartikel in der Dispersion im Bereich von 10 bis 500 nm, vorzugsweise 20 bis 200 nm und noch bevorzugter 50 bis 150 nm liegt.

10. Zusammensetzung nach einem der Ansprüche 1 bis 9, wobei das Copolymer von Methacrylsäure und C₁₋₄-Alkylacrylat das vernetzte Methacrylsäure/Ethylacrylat-Copolymer in Form einer wässrigen Dispersion von 30 % ist.

11. Zusammensetzung nach einem der Ansprüche 1 bis 9, wobei das Copolymer von Methacrylsäure und C₁₋₄-Alkylacrylat in einer Konzentration von 0,01 bis 0,4 Gew.-% wirksame Substanz, bezogen auf das Gesamtgewicht der Zusammensetzung, und vorzugsweise 0,1 bis 0,3 Gew.-% enthalten ist.

12. Zusammensetzung nach einem der Ansprüche 1 bis 11, wobei der Elektrolyt unter den Alkalimetallsalzen ausgewählt ist.

13. Zusammensetzung nach Anspruch 12, wobei der Elektrolyt das Natriumchlorid, Kaliumchlorid oder Kaliumsulfat ist.

14. Zusammensetzung nach einem der Ansprüche 1 bis 13, wobei der Elektrolyt in Konzentrationen größer oder gleich 0,25 %, bezogen auf das Gesamtgewicht der Zusammensetzung, und insbesondere 0,5 bis 3 Gew.-% enthalten ist.

15. Zusammensetzung nach Anspruch 14, wobei der Elektrolyt in Konzentrationen von 0,25 bis 5 Gew.-%, bezogen auf das Gesamtgewicht der Zusammensetzung, und insbesondere 0,5 bis 3 Gew.-% enthalten ist.

16. Zusammensetzung nach einem der Ansprüche 1 bis 15, wobei der anionische grenzflächenaktive Stoff unter den Carboxylaten, Alkylsulfaten, die gegebenenfalls ethoxyliert sind; Sulfonaten; Alkylsulfoacetaten; Phosphaten; Polypeptiden; anionischen Derivaten von Alkylpolyglucosid und deren Gemischen ausgewählt ist.

17. Zusammensetzung nach Anspruch 16, wobei der anionische grenzflächenaktive Stoff unter den Salzen von Alkylethersulfaten mit 6 bis 24 Kohlenstoffatomen ausgewählt ist, die 1 bis 30 Ethylenoxidgruppen aufweisen.

18. Zusammensetzung nach Anspruch 17, wobei der anionische grenzflächenaktive Stoff unter den ethoxylierten Natriumalkyl(C₁₂₋₁₄)ethersulfaten, die eine mittlere Zahl an Ethylenoxidgruppen von 1 bis 4 aufweisen, und insbesondere Sodium Laureth Sulfate ausgewählt ist.

19. Zusammensetzung nach einem der Ansprüche 1 bis 18, die ferner mindestens einen amphoteren oder zwitterionischen grenzflächenaktiven Stoff enthält.

20. Zusammensetzung nach Anspruch 19, wobei der amphotere oder zwitterionische grenzflächenaktive Stoff unter den Alkylbetainen, N-Alkylamidobetainen und deren Derivaten, Sultainen, Alkylpolyaminocarboxylaten, Alkylamphoacetaten und deren Gemischen ausgewählt ist.

21. Zusammensetzung nach Anspruch 20, wobei der amphotere oder zwitterionische grenzflächenaktive Stoff ausgewählt ist unter
- Cocobetain,
- Dinatriumcocoamphodiacetat oder deren Gemischen.

22. Zusammensetzung nach einem der Ansprüche 1 bis 21, die ferner mindestens einen nichtionischen grenzflächenaktiven Stoff enthält.

23. Zusammensetzung nach Anspruch 22, wobei der nichtionische grenzflächenaktive Stoff ausgewählt ist unter
- Alkoholen, Alphadiolen, Alkylphenolen oder Fettsäuren, die polyethoxyliert, polypropoxyliert oder mehrfach mit Glycerin verethert sind und eine Fettkette aufweisen ,
- Copolymeren von Ethylenoxid und Propylenoxid,
- Kondensaten von Ethylenoxid und Propylenoxid mit Fettalkoholen,
- polyethoxylierten Fettamiden,
- mehrfach mit Glycerin veretherten Fettamiden ,
- polyethoxylierten Fettaminen,
- ethoxylierten Sorbitanfettsäureestern,
- Saccharosefettsäureestern,
- Polyethylenglycolfettsäureestern,
- Alkyl(C₆₋₂₄)polyglycosiden,
- N-Alkyl(C₆₋₂₄)glucaminderivaten,
- Aminoxiden,
- N-Acyl(C₁₀₋₁₄)aminopropylmorpholinoxiden
und deren Gemischen.

24. Zusammensetzung nach einem der Ansprüche 1 bis 22, wobei die Gesamtmenge des grenzflächenaktiven Stoffes (oder der grenzflächenaktiven Stoffe), ausgedrückt als Gewicht der wirksamen Substanz, im Bereich von 5 bis 50 Gew.-%, vorzugsweise 6 bis 50 Gew.-%, besser 6 bis 30 Gew.-% und noch besser 8 bis 25 Gew.-%, bezogen auf das Gesamtgewicht der Zusammensetzung, liegt.

25. Zusammensetzung nach einem der Ansprüche 1 bis 24, die ferner ein oder mehrere kationische Polymere enthält.

26. Zusammensetzung nach einem der Ansprüche 1 bis 25, die ferner einen oder mehrere kosmetische Zusatzstoffe enthält, die unter den Parfums, Konservierungsmitteln, Maskierungsmitteln (EDTA), Pigmenten, Perlglanzstoffen oder Trübungsmitteln, anorganischen oder organischen Füllstoffen, Mattierungsmitteln, Bleichmitteln oder exfoliativen Stoffen, löslichen Farbstoffen, kosmetischen oder dermatologischen Wirkstoffen, nichtionischen Polymeren, anionischen Polymeren und Fettsubstanzen ausgewählt sind.

27. Kosmetische Verwendung einer Zusammensetzung nach einem der Ansprüche 1 bis 26 als Produkt für die Reinigung und/oder zum Abschminken von menschlichen Keratinsubstanzen.

28. Verwendung nach Anspruch 27 als Produkt für das Bad oder die Dusche.

29. Verwendung nach Anspruch 27 als Produkt für die Reinigung der Hände.

30. Verwendung nach Anspruch 27 als Haarwaschmittel.

31. Verwendung nach Anspruch 27 als Produkt zum Abschminken der Augen, des Gesichts oder der Lippen.

32. Verwendung der Zusammensetzung nach einem der Ansprüche 1 bis 26 für die Herstellung einer Formulierung, die für die Behandlung von fettiger Haut vorgesehen ist.

33. Kosmetisches Verfahren für die Reinigung von Schmutzrückständen an menschlichen Keratinsubstanzen, **dadurch gekennzeichnet, dass** eine Zusammensetzung nach einem der Ansprüche 1 bis 26 auf die Keratinsubstanzen in Gegenwart von Wasser aufgebracht wird, zur Bildung eines Schaums massiert wird und der gebildete Schaum und die Schmutzrückstände durch Spülen mit Wasser entfernt werden.

34. Verwendung eines vernetzten Copolymers von Methacrylsäure und C₁₋₄-Alkylacrylat, wie es in einem der Ansprüche 1 bis 10 definiert ist, in einer Menge unter 0,5 Gew.-% wirksame Substanz in einer flüssigen Reinigungszusammensetzung, wie sie in den Ansprüchen 1 bis 26 definiert ist, um die Stabilität der Textur der Zusammensetzung bei einer Temperatur unter 20 °C zu verbessern.
